# EUROPÄISCHE PATENTSCHRIFT

(11) **EP 2 818 477 B1**
(45) Veröffentlichungstag und Bekanntmachung des Hinweises auf die Patenterteilung: **04.05.2016**
(21) Anmeldenummer: 12868924.7
(22) Anmeldetag: 18.05.2012
(51) Int. Cl.: C07K 5/09, A61K 38/06, A61P 3/06, A61P 3/10

(54) **L-LYSYL-L-GLUTAMYL-L-TRYPTOPHAN ZUR KORREKTUR DES STOFFWECHSELSYNDROMS**
L-LYSYL-L-GLUTAMYL-L-TRYPTOPHAN FOR CORRECTING METABOLIC SYNDROME
L-LYSYL-L-GLUTAMYL-L-TRYPTOPHANE POUR CORRIGER LE SYNDROME MÉTABOLIQUE

(43) Veröffentlichungstag der Anmeldung: 31.12.2014
(73) Patentinhaber: Obshestvo S Organichennoj Otvetstvennostju "CytoNIR", St. Petersburg 191023 (RU)
(72) Erfinder: MALININ, Vladimir Viktorovich, St.Peterburg 197227 (RU)
(74) Vertreter: Jeck, Anton
(86) Internationale Anmeldenummer: PCT/RU2012/000261
(87) Internationale Veröffentlichungsnummer: WO 2013/172728

(56) Entgegenhaltungen:
- EP-A1- 0 818 462
- WO-A1-2011/017799
- CA-A1- 2 276 542
- RU-C1- 2 107 691
- RU-C1- 2 458 935
- DATABASE WPI Week 200138 Thomson Scientific, London, GB; AN 2001-365638 XP002733445, & RU 2 165 767 C1 (CHERNOV YU N) 27. April 2001 (2001-04-27)

## Beschreibung

Die Erfindung betrifft ein Mittel zur Korrektur des metabolischen Syndroms.

Die Erfindung ist in der Medizin, nämlich bei Peptidverbindungen und insbesondere bei Tripeptiden, als Mittel zur Korrektion der Störungen von Lipid- und Kohlenhydratstoffwechsel des metabolischen Syndroms einsetzbar.

Das metabolische Syndrom, Syndrom X) ist eine Gesamtheit von hormonalen und metabolischen Störungen der zusammenhängenden cardio-vasculären und Diabetes-Risikofaktoren, die von einem gemeinsamen pathophysiologischen Mechanismus, und zwar Insulinresistenz, vereinigt werden. Das metabolische Syndrom setzt sich aus mehreren "Bestandteilen" zusammen und zwar: abdominelle viszerale Fettleibigkeit, Insulinresistenz und Hyperinsulinismus, gestörte Glukosetoleranz, Früharteriosklerose, Dyslipidämie, Mikroalbuminurie, Purinstoffwechsel-Pathologie. So gut wie alle Partner des metabolischen Syndroms tragen dazu bei und sind unabhängige cardiovasculäre Risikofaktoren, und die Kombination mehrerer Partner vergrößert die Gefährdung ihrer Entwicklung mehrfach.

Das Problem der Behandlung des metabolischen Syndroms steht im Fokus der praktischen Ärzte. Hauptziel der Behandlung der vom metabolischen Syndrom betroffenen Patienten ist eine möglichst große Risikosenkung hinsichtlich der cardivasculären Erkrankungen sowie ihrer gefährlichen Komplikationen. Die Wirksamkeit der medikamentösen Behandlung des metabolischen Syndroms wird vom Standpunkt der Vorbeugung der mit Arteriosklerose zusammenhängenden Krankheiten sowie ihrer Entwicklung und Komplikationen beurteilt. Die Korrektion der Insulinresistenz als pathophysiologisches Kettenglied des metabolischen Syndroms stellt ebenfalls einen wichtigen Bereich der Pharmakotherapie dar [V.I. Malkin. Das metabolische Syndrom. - M.: Informationsagentur für Medizin, 2010.-144 S.].

Eine Möglichkeit, das metabolische Syndrom abzugleichen, könnte eventuell die Entwicklung der genannten Krankheiten verhindern. Jedoch sind Mittel für eine umfassende allgemeine Korrektion des metabolischen Syndroms bis jetzt nicht bekannt.

Es ist Aufgabe der Erfindung, eine Korrektur der Störungen von Lipid- und Kohlenhydratstoffwechsel beim Metabolischen Syndrom, d. h. einer MS-Korrektion, zu entwickeln.

Die gestellte Aufgabe wird dadurch gelöst, dass als Mittel zur Korrektur des metabolischen Syndroms Tripeptid L-Lysyl -L-Glutamyl-L-Tryptophan mit allgemeiner Formel 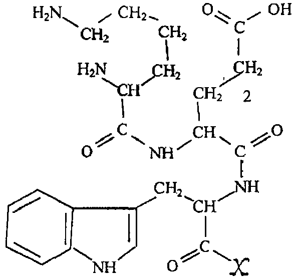 verwendbar ist, wobei X = OH oder NH₂ ist.

Die Verbindungen können als Lys-Glu-Trp (X=OH) und Lys-Glu-Trp-NH2 bezeichnet werden.

Lys-Glu-Trp-OH (X=OH) ist in CA 2276542(Anspruch 2) als Mittel zur Hämopoese-Modulation bei Tieren erwähnt. Der Einsatz zwecks MS-Korrektion ist in der Literatur nicht beschrieben.

Das Tripeptid ist nach dem bekannten Verfahren von Peptidsynthese in einer Lösung erzeugt [Yakubke H.D. Eshkayt H. Aminosäuren, Peptide, Proteine: Übersetzung aus dem Deutschen - M.: Mir, 1985, 456 S.; Ovchinnikov Yu.A. Bioorganische Chemie. - M.: Prosveschenie, 1987. - 815 S.].

Der Grundsubstanzgehalt ist nach dem Verfahren einer Hochdruckflüssigkeitschromatographie am "Beckman"-Chromatographen in Säule Vydac C18, 218TP, 4,6 x 250 mit folgenden Daten ermittelt: Fließmittel 0,05 % Trifluoressigsäurelösung in Azetonitril, Gradient 23 to 48 % innerhalb von 25 Minuten, Strömungsgeschwindigkeit 1,0 ml/Min., Erkennung bei 220 nm, Grundsubstanz-gehalt 95,9 % (X = NH₂) und 96,9 % (X = OH). Das berechnete Molekulargewicht ist 460,53 (X = NH₂) und 461,51 (X = OH). Das gemessene Molekulargewicht ist jeweils 460,98 und 462,05. Die allgemein toxische Wirkung von Peptid gemäß der erfindungsgemäßen Verbindung ist in Übereinstimmung mit den Vorschriften des "Handbuchs für experimentelle (präklinische) Forschung von neuen pharmakologischen Stoffen" (2005) erforscht: Es handelte sich um eine akute Toxizität bei einmaliger Verabreichung des Präparats sowie um eine subakute und chronische Toxizität bei andauernder Peptid-Verabreichung.

Die akute Toxizität ist an 60 weißen Mäuse-Männchen (Outbred mice) mit einem Körpergewicht von 18 - 20 g erforscht.

Die subakute Toxizität ist an 50 weißen rasselosen Ratten-Männchen mit einem Körpergewicht von 180 - 220 g erforscht. Die morphologische Zusammensetzung und die Eigenschaften des peripheren Bluts wurden vor der Verabreichung des Präparats sowie am 30., 60. und 90. Tag ab Anfang der Behandlung der Tiere mit diesem Präparat untersucht. Nach Abschluss des Experiments wurden die bio-chemischen und die Koagulation-Blutkenndaten studiert.

Die Forschungen der chronischen Toxizität wurden im Laufe von 6 Monaten durchgeführt. Diese Dauer ist aufgrund einer empfohlenen klinischen Verordnung des Präparats festgelegt. Die Forschung wurde an 76 Meerschweinchen-Männchen mit einem Körpergewicht von 290 - 320 g durchgeführt. Folgende Kenndaten des peripheren Bluts der Tiere wurden anhand allgemein anerkannter Verfahren bestimmt: Menge Erythrozyten, Hämoglobingehalt, Retikulozytengehalt, Thrombozytengehalt, Leukozytengehalt, Differentialblutbild, Blutsenkungsgeschwindigkeit (BSG) und Erythrozytenresistenz. Daneben wurden auch der Gesamteiweißgehalt nach dem Lowry-Verfahren sowie der Kalium- und Natriumgehalt im Blutserum nach dem Plasma-Spektralfotometrieverfahren ermittelt. Nach Abschluss des Versuchs wurde eine pathomorphologische Untersuchung von Hirn und Mark, Spinalganglien, Schilddrüse, Parathyreoidkörper, Nebennieren, Orches, Hypophyse, Herz, Lungen, Aorta, Leber, Nieren, Harnblase, Pankreas, Magen, Dünndarm, Dickdarm, Thymus, Milz, Lymphknoten und Knochenmark durchgeführt.

Während der Untersuchung der akuten Toxizität wurde Folgendes festgestellt. Die einmalige Verabreichung des untersuchten Peptids der Tiere in einer Menge, die die therapeutische für die klinische Verwendung empfohlene Dosis mehr als das 5000-fache überschreitet, ruft keine toxischen Reaktionen hervor. Das zeugt von einem großen therapeutischen Wirkungsspektrum des Präparats.

Die Untersuchung der subakuten und chronischen Toxizität von Peptid weist auf keine Nebeneffekte bei einer Daueranwendung des Präparats in 100- bis 1000-fach höheren Dosen hin, als es therapeutisch vorgesehen ist. Während der Untersuchung sind keine gesicherten Auswirkungen des erforschten Peptids auf die morphologischen und biochemischen Kennwerte des peripheren Bluts sowie auf BSG und die Erythrozytenresistenz festgestellt worden.

Bei der Beurteilung des allgemeinen Zustands der Tiere sowie bei Auswertung von morphologischen und biochemischen Kennwerten des peripheren Bluts, des morphologischen Zustands der Innenorgane, des Zustands des Herz-Kreislaufsystems und des Atemsystems, der Funktionsfähigkeit der Nieren und der Leber sind keine pathologischen Veränderungen im Körper der Tiere erkennbar.

Die pharmakologische Wirkung der erfindungsgemäßen Substanz ist an Versuchsmodellen von Hyperlipidämie, alimentärer atherogener Dyslipoproteinämie und Alloxan-Diabetes sowie klinisch bei der Behandlung eines metabolischen Syndroms bei älteren Patientengruppen erforscht.

Die Untersuchungsergebnisse der pharmakologischen Wirkung der erfindungsgemäßen Substanz sind in Beispielen angeführt. Um die Darlegung der Information zu vereinfachen, ist die erfindungsgemäße Verbindung basisch (X = OH) als Lys-Glu-Trp und in Amid-Form (X = NH₂) als Lys-Glu-Trp-NH₂ bezeichnet.

### Beispiel 1. Einfluss der erfindungsgemäßen Tripeptide auf den Lipidspiegel bei Ratten im Versuchsmodell der durch Polyoxyethylen-orbitan-mono-oleat verursachten Hyperlipidämie

Die Versuche wurden an 51 Outbred-Ratten-Männchen der Linie Sprague-Dawley mit Körpergewicht von 220 - 240 g durchgeführt.

Die untersuchten Peptide wurden täglich in einer Tagesdosis von 20 und 200 µg/kg in den Magen im Laufe von 5 Tagen bis Hyperlipidämie-Ansatz verabreicht. Als Vergleichspräparat wurde ein Präparat gegen Hyperlipidämie aus der Gruppe von Fibric-Säure-Derivaten oder Fibraten - Bezafibrat (2-(b-parachlorobenzoyl-aminoethyl)phenoxy-2-methylpropinsäure) in der Dosis 50,0 mg/kg verwendet.

Die Hyperlipidämie wurde bei Ratten mittels intraperitonealer Einführung von Polyoxyethylen-sorbitan-monooleat (Polysorbat-80, Mischung von Polyäthylen-glykol-Esteren von Anhydrosorbit Monooleat) in einer Dosis von 300 mg/kg verabreicht. 24 Stunden später wurden die Ratten eingeschläfert. Danach wurden Triglyzeridspiegel, Gesamtcholesterin und Lipoproteid-Cholesterin mit hoher Dichte (HDL) im Blutserum der Tiere gemessen.

Infolge der Untersuchung der spezifischen pharmakologischen Aktivität wurde Folgendes festgestellt. Im Hintergrund der durch Polyoxyethylen-sorbitan-monooleat verursachten Hyperlipidämie üben Peptide eine hypolipidämische Wirkung aus. Das zeigt sich durch Senkung des Triglyzeridspiegels, des Gesamtcholesterinspiegels sowie durch Steigerung des HDL-Cholesterins im Blut (Tabelle 1) an.

**Tabelle 1**

| Einfluss der Tripeptide Lys-Glu-Trp und Lys-Glu-Trp-NH2 auf die Lipidstoff-wechsel-Kennziffern bei Ratten im Falle der durch Polyoxyethylen-sorbitan-monooleat verursachten Hyperlipidämie | | | |
|---|---|---|---|
| Versuchsgruppen / Dosis | Triglyzeridspiegel, mMol/l | Gesamtcholesterin, mMol/l | HDL- Cholesterin, mMol/l |
| Intakt- Gruppe | 0,66±0,03 | 1,16±0,04 | 1,01±0,04 |
| Referenzgruppe (Hyperlipidämie) | 1,29±0,05* | 2,77±0,20* | 0,55±0,04* |
| Tripeptid Lys-Glu-Trp, 20 µg/kg | 0,58±0,05** | 1, 55±0,20** | 0,97±0,04** |
| Tripeptid Lys-Glu-Trp, 200 µg/kg | 0,67±0,05** | 2,10±0,17** | 0,60±0,02* |
| Tripeptid Lys-Glu-Trp-NH2, 20 µg/kg | 0,76±0,14** | 1,69±0,15** | 1,05±0,02** |
| Tripeptid Lys-Glu-Trp-NH2, 200 µg/kg | 0,88±0,12** | 1,22±0,15** | 1,09±0,02** |
| Bezafibrat, 50,0 mg/kg | 0,55±0,06** | 1,34±0,03** | 0,98±0,05** |

| | | | |
|---|---|---|---|
| * - P<0,05 im Vergleich zu Intakttieren; ** - P<0,05 im Vergleich zu Tieren aus der Referenzgruppe. | | | |

### Beispiel 2. Einfluss von Tripeptid Lys-Glu-Trp auf den Lipidspiegel bei Ratten im Versuchsmodell mit alimentärer atherogener Dyslipoproteinämie

Der Versuch wurde an 40 weißen Ratten-Männchen der Wistar-Linie mit einem Körpergewicht von 230 - 250 g durchgeführt. Die Tiere wurden in 4 Gruppen aufgeteilt. Gruppe 1: Referenzgruppe (Intakt), Gruppen 2 bis 4: Versuchsgruppen.

Die Versuchstiere wurden auf hypercholesterinämische (HCÄ) Diät gesetzt, um eine alimentäre atherogene Dyslipoproteinämie (DLP) zu modellieren. Die HCÄ- Diät umfasste mind. 7,5 % Cholesterin (CS), 45 % Fette und einen Überschuss an Vitamin D₂ (500 000 IE). Gleichzeitig mit der Entwicklung von DLP erhielt die 3. Gruppe von Tieren Lys-Glu-Trp in einer Dosis von 100 µg/kg intraperitoneal täglich. Die 4. Gruppe von Tieren erhielt Lipanthyl 25 mg/kg intraperitoneal Lipanthyl 200M der Fa. Lab.Fournier S.A. (Frankreich) oder Fenofibrate, die zur Behandlung von Hyperlipoproteinämie verwendet werden, die sich mit keiner Diät mehr behandeln lässt. Der Versuch dauerte 24 Tage. Während des Versuchs hatten die mit Lys-Glu-Trp und Lipanthyl behandelten Tiere normgerechtes Aussehen. Sie waren wohlgenährt und hatten glattes glänzendes Fell. Die Tiere aus der 2. Gruppe hatten zerzaustes Fell und zeigten Übererregbarkeit, was durch einen Überschuss an Vitamin D₂ erklärt werden kann.

Nach dem Versuchsende wurde eine Dekapitation der Tiere vorgenommen. Danach wurden der Gesamt-Cholesterinspiegel (CS), Triglyzeridspiegel (TG), Cholesteringehalt der Lipoproteine hoher Dichte (CS HDL) im Blutserum der Tiere beurteilt und die Cholesterin-Konzentration der Lipoproteine niedriger Dichte (CS LDL) sowie der Cholesterin-HDL Quotient (LDL/HDL-Verhältnis) berechnet. Die genannten Kennziffern sind der Tabelle 2 zu entnehmen.

**Tabelle 2**

| Einfluss von Tripeptid Lys-Glu-Trp auf Lipidstoffwechsel-Kennwerte unter alimentärer DLP, die durch Rattenernährung nach einer HCÄ-Diät im Laufe von 24 Tagen hervorgerufen wurde | | | | | | |
|---|---|---|---|---|---|---|
| Nr. | Gruppe von Tieren | Triglyzeridspiegel (mMol/l) | CS (mMol/l) | CS HDL (mMol/l) | CS LDL (mMol/l) | LDL/HDL-Verhältnis |
| 1 | Intakt- Gruppe | 0,64±0,05 | 0,97±0,10 | 0,70±0,05 | -0,02±0,11 | 0,40±0,19 |
| 2 | HCÄ Diät | 1,59±0,18* | 3,35±0,22* | 0,31 ±0,07* | 2,32±0,20* | 11,0±2,7* |
| 3 | HCÄ Diät + Tripeptid Lys-Glu-Trp | 0,96±0,16** | 1,70±0,27* * | 0, 55±0,10** | 0,71±0,24** | 2,21±0,58* * |
| 4 | HCÄ + Lipanthyl | 0,62±0,08** | 2,24±0,21* * | 0,55±0,08** | 1,41 ±0,22** | 3,25±0,75* * |

| | | | | | | |
|---|---|---|---|---|---|---|
| * - P<0,05 im Vergleich zum Kennwert für Intakttiere (1. Gruppe); ** - P<0,05 im Vergleich zum Kennwert für Tiere nach HCÄ Diät (2. Gruppe). | | | | | | |

Bei der Beurteilung wurden der Zustand und das Gewicht von Leber, Milz und Nebennieren berücksichtigt.

Aus der Tabelle 2 geht hervor, dass ein Lys-Glu-Trp alle Kennwerte, die auf die Entwicklung von alimentärer DLP hinweisen, zuverlässig sinken lässt.

Lipanthyl vermindert ebenfalls die Kennwerte, welche von der Entwicklung der alimentären DLP zeugen. Jedoch ist die Verminderung des LDL/HDL-Verhältnisses kleiner als bei einer Behandlung mit Lys-Glu-Trp.

Die Untersuchungen der Innenorgane der Tiere ergaben, dass Lys-Glu-Trp keine ungünstige Wirkung auf das relative Gewicht von Leber, Milz und Nebennieren hatte. Lipanthyl wirkte der Zunahme von sowohl absoluter als auch relativer Lebermasse nicht entgegen.

Somit wurde eine hypolipidämische Wirkung von Tripeptid am Versuchsmodell alimentärer DLP veranschaulicht. Diese Wirkung ist mit dem Effekt des bekannten Präparats aus der Fibrat-Gruppe vergleichbar bzw. übertrifft ihn. Dabei rief die Gabe von Tripeptid keine Nebenwirkungen und Komplikationen hervor.

### Beispiel 3. Einfluss von Insulin auf den Glukosegehalt im Blut der Ratten mit Alloxan-Diabetes nach einer Anwendung von Tripeptid Lys-Glu-Trp-NH₂

Bekanntlich ist Alloxan-Diabetes durch Schädigung der ß-Zellen von Pankreas gekennzeichnet und wird von ausgeprägter Hyperglykämie infolge eines Insulinmangels und einer Aktivierung von Glukoneogenese begleitet.

Der Versuch wurde an 18 weißen Outbred-Ratten-Männchen mit einem durchschnittlichen Körpergewicht von 349 ± 19 g durchgeführt. Nach der Ermittlung von Glukosekonzentration im Blut wurden alle Tiere in 2 Gruppen aufgeteilt. Danach erhielten alle Tiere einmalig intravenös je 1 ml Alloxanlösung ("Spofa") in einer Dosis von 35 mg/kg. 15 Tage danach wurde den Tieren aus der Referenzgruppe je 0,3 ml der 0,9 %igen NaCl-Lösung einmal täglich intraperitoneal verabreicht. Die Ratten aus der Hauptgruppe wurden mit Tripeptid Lys-Glu-Trp-NH₂ in einer Dosis von 3 µg (in 0,3 ml der 0,9 %igen NaCl-Lösung) pro Ratte innerhalb von 11 Tagen behandelt. Eine separate Gruppe bestand aus 7 gesunden Ratten mit einem ähnlichen Körpergewicht.

Es wurde festgestellt, dass die Einführung von Tripeptid Lys-Glu-Trp-NH₂ zu einer sicheren Senkung des Glukosespiegels im Blut der Tiere am 28. Tag der Forschung beitrug. Die Senkung betrug im Durchschnitt 34,8 %. Die Senkung des Glukosespiegels bei einer Behandlung mit Tripeptid Lys-Glu-Trp-NH₂ korrelierte mit einer Senkung der Letalität unter den Tieren der Hauptgruppe.

Danach wurde allen Tieren Insulin (0,3 ME) intravenös verabreicht und die Glukosekonzentration im Blut gemessen.

Die Ergebnisse des Versuchs sind in Tabelle 3 enthalten. Bei den gesunden Tieren wurde eine akute physiologische Senkung des Glukosespiegels beobachtet, während dieser Kennwert bei der Vergleichsgruppe (Alloxan-Diabetes) um das 2,7-fache niedriger war. Bei den mit Alloxan-Diabetes betroffenen und mit Tripeptid Lys-Glu-Trp-NH₂ behandelten Tieren wurde eine fast zweimal größere Glukosespiegelsenkung gegenüber der Vergleichsgruppe nach der Insulineinführung festgestellt. Diese Angaben weisen auf die Fähigkeit von Tripeptid Lys-Glu-Trp-NH₂ hin, die Empfindlichkeit der Gewebe gegen Insulin in hohem Maße aufrechtzuerhalten

**Tabelle 3**

| Einfluss von Insulin auf den Glukosegehalt im Blut der Ratten mit Alloxandiabetes nach einer Anwendung von Tripeptid Lys-Glu-Trp-NH₂ | | | |
|---|---|---|---|
| Gruppe von Tieren | Glukosekonzentration im Blut (mg %) | | Senkung von Glukosespiegel im Vergleich zum Ausgangsspiegel (%) |
| | Ausgangsspiegel | 30 Minuten nach Insulineinführung | |
| Gesunde | 83,5±3,2 | 29,4±2,2 | 64,8 |
| Referenzgruppe (Alloxandiabetes + NaCl) | 375,7±11,2* | 287,8±12,5* | 23,4 |
| Alloxandiabetes + Tripeptid Lys-Glu-Trp-NH2 | 221,5±11,2* | 123,6±8,3# | 44,2 |

| | | | |
|---|---|---|---|
| * - P < 0,001 im Vergleich zu gesunden Tieren; # - P < 0,05 im Vergleich zur Referenzgruppe. | | | |

Tripeptid Lys-Glu-Trp-NH₂ ruft keine Nebenwirkungen, Komplikationen und keine Arzneiabhängigkeit hervor.

### Beispiel 4. Effektivität der Anwendung von Tripeptid Lys-Glu-Trp-NH₂ bei einer Behandlung des metabolischen Syndroms bei älteren Patientengruppen

Die Untersuchung bezog sich auf Patienten mit einer verifizierten Diagnose eines metabolischen Syndroms. Die Diagnose wurde bei Erfüllung folgender Kriterien gestellt: arterielle Hypertension, gestörte Glukosetoleranz, Fettsucht, Dyslipidämie. Es wurden insgesamt 85 Patienten mit metabolischem Syndrom (50 Frauen, 35 Männer) untersucht. Das durchschnittliche Alter betrug bei den Männern 54,6 ± 12 Jahre, bei den Frauen 55,6 ± 13,0 Jahre.

Kriterien für die Einbeziehung der Patienten in die Forschung:
1. Alter: Älter als 45 Jahre für Männer und Frauen.
2. Vorhandensein von klinischen Merkmalen eines metabolischen Syndroms.
3. Vorhandensein von verifizierter Pathologie des Magen-Darm-Kanals (Verstopfungen, Reizdarmsyndrom, Cholezystitis, Pankreatitis, Fettleber).
4. Zustimmung des Patienten.

Die Kriterien für den Ausschluss der Kranken aus der Untersuchung waren das Vorhandensein von familiärer Dyslipidämie, akuter Pathologie des Magen-Darm-Kanals, Merkmalen der Helmintheninvasion und onkologischer und systematischer Pathologie.

Die Kombination der angewandten pathogenetischen Therapie unter Einsatz solcher Mittel wie ACE-Hemmer, Ca2⁺-Kanal-Blocker, harntreibende Mittel, β-Blocker und deren Dosierung wurde aufgrund klinischer Indikationen festgelegt. Bei der Untersuchung wurden auch die mit Nitropräparaten sowie mit Statinen behandelten Kranken ausgeschlossen. Alle Kranken wurden einer hypocholesterinämisierenden Diät unterzogen, die eine Einnahme von Kleie einschließt.

Tripeptid Lys-Glu-Trp-NH₂ wurde 47 Patienten oral in einer Dosis von 500 µg pro Gabe zweimal täglich im Laufe von 30 Tagen verabreicht. Die Unterbrechung zwischen den Behandlungen betrug 2 Monate.

Die Kranken wurden im Laufe von 12 Monaten beobachtet. Die Untersuchung umfasste 3 Phasen: Ursprungsphase (zum Zeitpunkt der Diagnoseüberprüfung), 6 Monate und ein Jahr später.

Folgende Parameter wurden bei allen Kranken analysiert: Geschlecht, Alter zum Zeitpunkt der Untersuchung, Krankheitsdauer, Dauer der arteriellen Hypertension, Fettsucht, Dyslipidämie. Die Untersuchung der Kranken umfasste eine Einschätzung der Risikofaktoren, wie z. B. Vererbung, Fettsucht, Zuckerkrankheit, koronare Herzkrankheit. Dabei wurde eine Sozialanamnese, d. h. Besonderheiten des Lebensstils, Ernährungsgewohnheiten, analysiert. Es wurden auch anthropometrische Messungen durchgeführt: Wuchs, Körpergewicht, Body-Maß- Index. Alle Kranken wurden einer planmäßigen Laboruntersuchung unterzogen und zwar: klinische Blutanalyse und Untersuchung von biochemischen Blutkennwerten. Es wurden Hämoglobingehalt, Erythrozytenmenge, Leukozyten-gehalt, Lymphozytengehalt ermittelt. Unter den biochemischen Serumkennwerten wurden Gesamteiweißgehalt, Albumine, Triglyzeridspiegel, Cholesterinspiegel, LDL, HDL, LDL/HDL-Verhältnis ermittelt. Die durchgeführten Instrumentenunter-suchungen umfassten Zuckerbelastungstest, Abdomen-Sonographie, Stuhluntersuchungen, Schilddrüsenfunktionsprüfung (Hormonstatus).

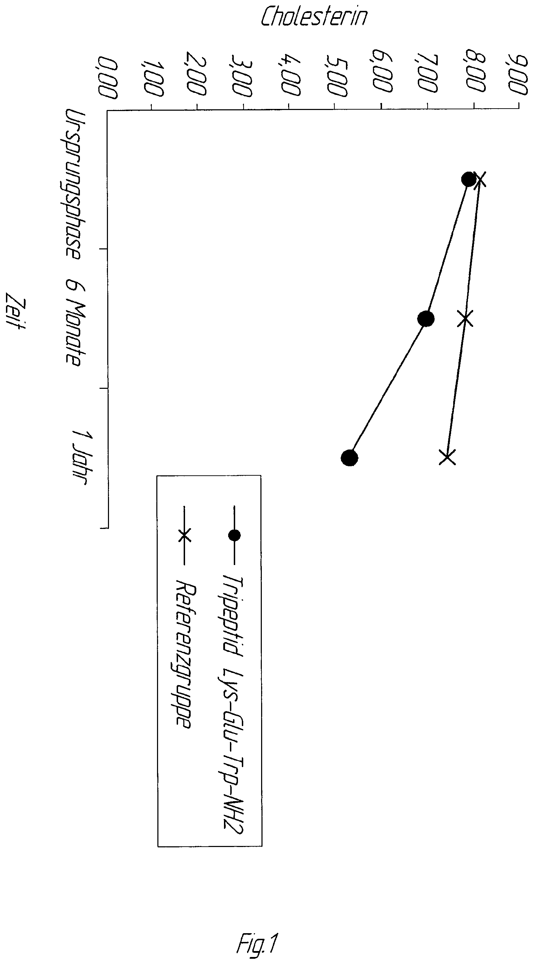

Die Ergebnisse der Anwendung von Lys-Glu-Trp-NH₂ bei Behandlung der MS- Erscheinungen sind in den Fig. 1 bis 3 dargestellt.

Es wurde infolge der durchgeführten Untersuchung im Rahmen der dynamischen Beobachtung festgestellt, dass die Anwendung von Tripeptid Lys-Glu-Trp-NH₂ zur Cholesterin-Konzentrationssenkung beitrug (s. Fig. 1).

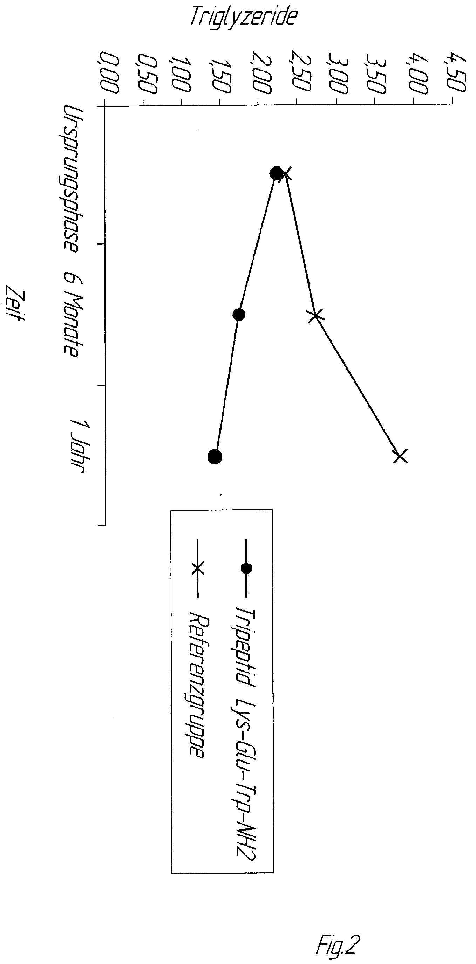

Unter Einwirkung von Tripeptid Lys-Glu-Trp-NH₂ wurde eine maximale Konzentrationssenkung der Triglyzeride für den Zeitraum der 3. Untersuchung nach Ablauf von einem Jahr (s. Fig. 2) beobachtet.

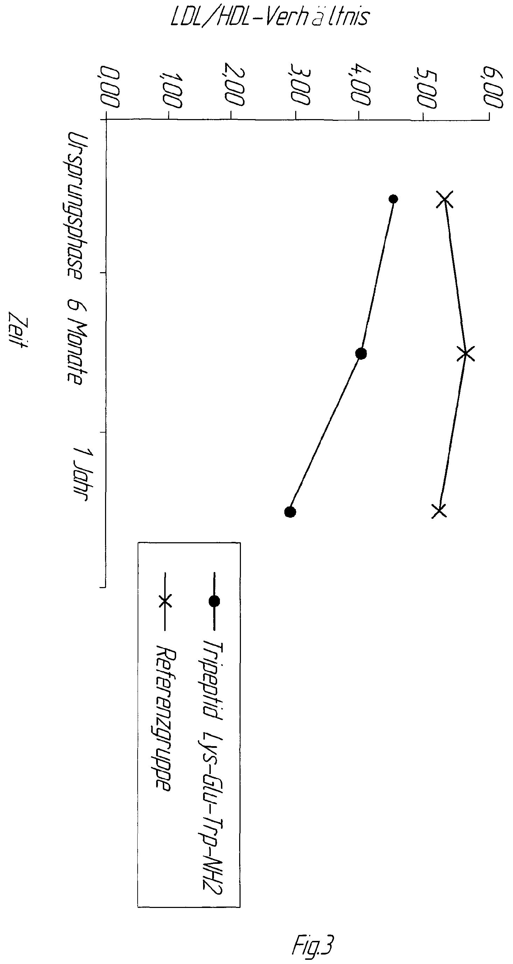

Im Hintergrund der Anwendung von Tripeptid Lys-Glu-Trp-NH₂ wurde auch eine ausgeprägte Verminderung des LDL/HDL-Verhältnisses beobachtet (s. Fig. 3).

Es wurde ein maßgeblicher Einfluss von dem bei Komplextherapie verwendeten Tripeptid Lys-Glu-Trp-NH₂ auf die Konzentrationssteigerung der Lipoproteine mit hoher Dichte erkannt.

Der Einsatz von Tripeptid Lys-Glu-Trp-NH₂ bei der Komplextherapie des metabolischen Syndroms leitete einen schnelleren und stabileren drucksenkenden Effekt ein.

Tripeptid Lys-Glu-Trp-NH₂ ruft keine Nebenwirkungen, Komplikationen und Arzneiabhängigkeit hervor.

Somit führte die Anwendung von Tripeptid Lys-Glu-Trp-NH₂ in Kombination mit der Basisbehandlung zur Verbesserung des Lipidspektrums, der Potenzierung des drucksenkenden Effekts sowie zur Verbesserung des Ernährungszustands der Patienten mit einem metabolischen Syndrom. Das bestimmt die Zweckmäßigkeit seiner weiten Anwendung in der therapeutischen und insbesondere in der geriatrischen Praxis.

Lysylglutamyltryptophan sowohl in basischer Form (X = OH) als auch in Amin-Form (X = NH2) kann in pharmazeutischen und chemischen Betrieben hergestellt werden, die sich auf organische Synthese spezialisiert haben.

## Patentansprüche

1. Mittel zur Verwendung in einer Behandlung zur Korrektur des metabolischen Syndroms, welches ein Tripeptid L-Lysyl -L-Glutamyl-L-Tryptophan mit der allgemeinen Formel 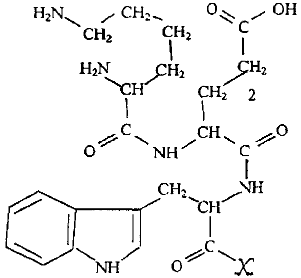 umfasst, wobei X = OH oder NH₂ ist.

2. Mittel zur Verwendung nach Anspruch 1, wobei der Lipidstoffwechsel beeinflusst wird.

3. Mittel zur Verwendung nach Anspruch 1, wobei der Kohlehydratstoffwechsel beeinflusst wird.

4. Mittel zur Verwendung nach einem oder mehreren der Ansprüche1 bis 3, zur Behandlung von Hyperlipidämie oder alimentärer atherogener Dyslipoproteinämie.

5. Mittel zur Verwendung nach einem oder mehreren der Ansprüche 1 bis 3 zur Behandlung des metabolische Syndroms bei älteren Patienten.

## Claims

1. Composition for use in a treatment for the correction of the metabolic syndrome, which comprises a tripeptide of L-lysyl-L-glutamyl-L-tryptophan with the general formula 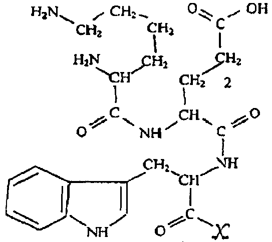 where X = OH or is NH₂.

2. Composition for use according to claim 1, wherein the lipid metabolism is influenced.

3. Composition for use according to claim 1, wherein the carbohydrate metabolism is influenced.

4. Composition for use according to one or more of the claims 1 to 3, for the treatment of hyperlipidaemia or alimentary atherogenic dyslipoproteinemia.

5. Composition for use according to one or more of the claims 1 to 3 for the treatment of the metabolic syndrome in the elderly.

## Revendications

1. Produit pour une utilisation dans un traitement destiné à la correction du syndrome métabolique, qui comprend un tripeptide L-lysyl-L-glutamyl-L-tryptophane de formule générale 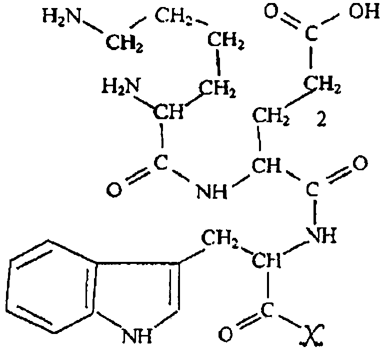 dans laquelle X = OH ou NH₂.

2. Produit pour l'utilisation selon la revendication 1, qui influe sur le métabolisme des lipides.

3. Produit pour l'utilisation selon la revendication 1, qui influe sur le métabolisme des glucides.

4. Produit pour l'utilisation selon l'une ou plusieurs des revendications 1 à 3, pour le traitement de l'hyperlipidémie ou de la dyslipoprotéinémie athérogène alimentaire.

5. Produit pour l'utilisation selon l'une ou plusieurs des revendications 1 à 3, pour le traitement du syndrome métabolique chez les patients âgés.
